(19) Europäisches Patentamt
European Patent Office
Office européen des brevets

(11) EP 3 791 790 B1

(12) **EUROPEAN PATENT SPECIFICATION**

(45) Date of publication and mention of the grant of the patent:
**26.07.2023 Bulletin 2023/30**

(21) Application number: **20188581.1**

(22) Date of filing: **30.07.2020**

(51) International Patent Classification (IPC):
***A61B 5/268*** *(2021.01)* ***A61N 1/04*** *(2006.01)*

(52) Cooperative Patent Classification (CPC):
**A61N 1/0484; A61B 5/268;** A61B 2562/125

(54) **SOFT MULTI-SEGMENTED, FUNCTIONALIZED BODIES MADE FROM POLYMERS**

WEICHE MEHRSEGMENTIERTE FUNKTIONALISIERTE KÖRPER AUS POLYMEREN

CORPS SOUPLES, MULTI-SEGMENTÉS ET FONCTIONNALISÉS FABRIQUÉS À PARTIR DE POLYMÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2019 US 201962881628 P**

(43) Date of publication of application:
**17.03.2021 Bulletin 2021/11**

(73) Proprietor: **Nextsense, Inc.**
**Mountain View, CA 94040 (US)**

(72) Inventors:
- **Dittmer, Robert**
  **63450 Hanau (DE)**
- **Stoica, Leonard**
  **63450 Hanau (DE)**
- **Hendricks, Jeffrey**
  **St. Paul, MN 55127 (US)**

(74) Representative: **Forresters IP LLP**
**Skygarden**
**Erika-Mann-Straße 11**
**80636 München (DE)**

(56) References cited:
**EP-A1- 3 400 865**
**WO-A1-2012/041507**
**WO-A1-2019/129391**
**US-B2- 9 682 225**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 791 790 B1

## Description

The present invention relates to a body, comprising at least one first polymer segment and at least one further polymer segment, wherein these segments are in seamless contact and differ with respect to their porosity and their electrical conductivity. The invention also relates to a process for the preparation of a body.

Skin surface electrodes are important components in many medical diagnostic systems including, for example, electrocardiography (ECG), electromyography (EMG), and electroencephalography (EEG). In these systems, the electrode plays a critical role as a transducer converting physiological variables, such as those of the heart, muscles and brain, respectively, in ECG, EMG and EEG, to electrical potentials (sometime referred to as biopotentials). The measured potentials are then amplified and processed by an instrument, such as an electronic measuring circuit and a computer. The workings of skin surface electrodes are dependent on a multitude of mechanisms, such as electrode material, the electrolyte applied to the electrode, body location, and skin properties. The electrode must convert a physiological ionic current into an electronic current receivable and readable by the associated instrumentation.

State of the art in the field of current-carrying and/or dissipating body electrodes optimized for short-term applications are systems that are based on aqueous gel electrolytes, such as those disclosed in US 9,050,451 B1. As an alternative for long-term applications electrodes are known in the prior art that are based on polymers comprising carbon, such as the graphite loaded polymers disclosed in US 7,136,691 B2, or electrodes that are based on conductive liquid elastomers filled with metallic particles, such as the rubber compositions disclosed in DE 10 2008 050 932 A1. It is also known in the prior art that doped thiophenes such as PEDOT/PSS can be used as conductive materials in body electrodes. Leleux et al. (Adv. Healthcare Mater, 2014, 3, pages 1377-1380) disclose ionic liquid gel-assisted electrodes for long-term cutaneous recordings, in which an ionic liquid gel was incorporated onto electrodes made of Au and PEDOT/PSS. These electrodes were deposited on a thin film of parylene C to render them conformable to the skin.

However, the electrodes known from the prior art, in which conductive materials such as PEDOT/PSS are simply coated onto a non-conductive support, are disadvantageous in several ways. First of all, for certain applications multi-channel electrodes are required in which two or more electrodes have to be accommodated in close neighborhood to each other, which by means of the prior art approaches for forming electrodes are often difficult to produce. Furthermore, skin surface electrodes often also must be characterized by a certain softness of the material that allows an optimized fit of the electrode, for example, in the ear of a patient. Also, if the electrode pathway is applied onto the surface of a non-conductive support, it is difficult to realize a sufficient robustness of the electrode, particularly in case of a multi-channel electrode.

US 9,682,225 B2 discloses a polymer film electrode comprising an electrically-conductive substrate and an electrically-conductive polymer applied to said electrically-conductive substrate, wherein said electrically-conductive polymer is in electrical contact with said electrically-conductive substrate, and said electrode exhibits ohmic behavior over a range of about 800 Hz to about 100 kHz.

WO 2019/129391 A1 discloses a pressure sensing layer comprising a film comprising a first layer of porous matrix material comprising a siloxane polymer, comprising a closed porosity volume fraction, and, optionally, an open porosity volume fraction, and a conductive or semiconductive filler substantially present in said closed porosity volume fraction of said first layer of porous matrix material and, optionally one or more additional layers.

EP 3 400 865 A1 discloses a conductive polymer composite based sensor, comprising a substrate and a sensor material, wherein the sensor material comprises an insulating polymer matrix component and an electrically conductive component dispersed in the polymer matrix component to form the conductive polymer composite, wherein the sensor material is pre-strained, and wherein the sensor material is applied to the substrate to form the sensor.

WO 2012/041507 A1 discloses a process for the production of a layer composition with an electrically conductive layer, comprising the process steps of providing a substrate with a substrate surface, forming a polymer layer comprising an electrically conductive polymer on at least a part of the substrate surface and applying a liquid stabilizer phase, comprising a stabilizer and a liquid phase, onto the polymer layer.

The present invention was based on the object of overcoming the disadvantages resulting from the prior art in connection with skin surface electrodes.

In particular, the present invention was based on the object of providing a body that can, for example, be used as a skin surface electrode, and which combines the advantages of high elasticity and high robustness.

A contribution to at least one of the above objects is given by the independent claims. The dependent claims provide preferred embodiments of the present invention which also serve solving at least one of the above mentioned objects.

The invention is defined in the claims. In particular, the invention relates to:
a body (100), comprising

i) at least one first polymer segment (101) having a porosity p1 and an electrical conductivity c1, and
ii) at least one further polymer segment (102) being in seamless contact with the at least one first polymer segment

(101) and having a porosity p2 ≠ p1 and an electrical conductivity c2 ≠ c1,

characterized in that the first polymer segment (101) has an electrical conductivity c1 of at least 0.001 S/cm and wherein the further polymer segment (102) has an electrical conductivity c2 of less than $1 \times 10^{-9}$ S/cm.

In an embodiment of the invention, p1 > p2.

In an embodiment of the invention, at least one segment selected from the first polymer segment (101) and the further polymer segment (102) is made from a conductive material that comprises a non-conductive polymer matrix and a conductive component dispersed therein.

In an embodiment of the invention, the at least one first polymer segment (101) has a compression modulus m1 and wherein the further polymer segment has a compression modulus of m2 ≠ m1, wherein the first polymer segment has a compression modulus m1 of less than 5000 MPa and wherein the at least one further polymer segment has a compression modulus m2 of at least 0.01 MPa.

Preferably, the conductive component comprises a conductive polymer. Preferably, the conductive polymer comprises polythiophene. Preferably, the polythiophene is a cationic polythiophene that is present in the form of a polythiophene/polyanion-complex. Preferably, the polythiophene/polyanion-complex is a PEDOT/PSS-complex.

In an embodiment of the invention, the further polymer segment (102) is made from a non-conductive material.

In an embodiment of the invention, the body (100) is comprised in a device selected from the group consisting of, or is a device selected from the group consisting of, a biosensor, a body-worn device having electrically sensing functionality and a body-worn device selected from the group consisting of a headphone, glasses, a headband, a chest-strap, an armband, a patch, a garment or a wrist-worn device.

The invention further relates to a process for the preparation of a body (100), comprising

I) preparing, by means of 3D-printing, at least one first polymer segment (101) having a porosity p1, preferably by using a first fill factor f1;
II) preparing, by means of 3D-printing, at least one further polymer segment (102) being in seamless contact with the at least one first polymer segment (101);
III) bringing into contact a conductive material with a segment selected from the first polymer segment (101) and the further polymer segment (102);

characterized in that the least one further polymer segment (102) has a porosity p2 ≠ p1, preferably by using a second fill factor f2 # f1.

In an embodiment of the invention, the body (100) is prepared in layers (109) by depositing droplets (104) of a molten polymer onto a surface of a substrate (105) or onto a surface of a previously applied layer (109), preferably onto the outer surface of a previously applied layer (109), wherein in the preparation of at least some of the layers (109) a first area (106) and a second area (107) are formed which differ with respect to the density in which the droplets (104) are deposited.

In an embodiment of the invention, at least one segment selected from the first poymer segment (101) and the further polymer segment (102) is made from a conductive material that comprises a non-conductive polymer matrix and a conductive component dispersed therein or at least one segment selected from the first polymer segment (101) and the further polymer segment (102) is made from a non-conductive material.

In an embodiment of the invention, at least one segment selected from the first polymer segment (101) and the further polymer segment (102) comprises void spaces (108) and wherein in process step III) a conductive component is introduced into at least some of the void spaces (108) or wherein in process step III) at least a part of the surface of at least one segment selected from the first polymer segment (101) and the further polymer segment (102) is coated with a conductive component.

The body according to the present invention preferably is a polymer body (i. e. a body which comprises at least 10 wt.-%, more preferably at least 25 wt.-% and most preferably at least 50 wt.-% of a polymer, preferably a thermoplastic polymer, based on the total weight of the body.

The body is characterized in that it comprises at least one first polymer segment and at least one further polymer segment, wherein these segments differ with respect to their porosity and their electrical conductivity and wherein these two segments are in seamless contact with each other. As used herein, the term *"in seamless contact"* is preferably understood in such a way that the material of the first segment and the material of the further segment flow into each other in the border area between these two segments (i. e. they form a coherent material). The first and the further segment are preferably not glued, welded of soldered to one another. Such a seamless contact is usually achieved when producing the first and further segment by means of 3D-printing in which these regions are produced by dropwise deposition of a molten thermoplastic material. In such a process drops which have been deposited to form the first segment come into contact with drops which have been deposited to form the further segment, wherein in the border region of the different segments the drops at least partially "fuse" and/or "entangle" with one another before they solidify

so that a seamless transition between the segments is obtained.

The term *"segment"* as used herein refers to any three-dimensional structure that may be a part of a complex object that has been obtained in a 3D-printing process. The object may thus have any dimensional shape such as a rod, a plate, a sphere, a pyramid, a cube, or any part-section or combination of these shapes.

Furthermore, a body in which the first and the segment differ with respect to both, their porosity p and their conductivity c, also comprises aspects in which one of these segments is not porous at all (i. e. is built from a completely solid mass that preferably does not comprise any voids at all) as well as aspects in which one of the segments does not show any practically useful electrical conductivity at all (i. e. is built from an insulating material).

<u>The conductive polymer</u>

The conductive polymer that may be comprised in the body according to the present invention or that in process step III) of the process according to the present invention may be brought into contact with a segment selected from the first polymer segment and the further polymer segment can be a polymer selected from the group consisting of an optionally substituted polythiophene, an optionally polyaniline and an optionally substituted polypyrrole, wherein optionally substituted polythiophenes are particularly preferred as the conductive polymer.

Preferred polythiophenes are those having the general formula

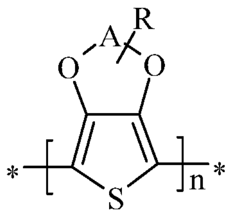

in which

A represents an optionally substituted $C_1$-$C_5$-alkylene radical,

R represents a linear or branched, optionally substituted $C_1$-$C_{18}$-alkyl radical, an optionally substituted $C_5$-$C_{12}$-cycloalkyl radical, an optionally substituted $C_6$-$C_{14}$-aryl radical, an optionally substituted $C_7$-$C_{18}$-aralkyl radical, an optionally substituted $C_1$-$C_4$-hydroxyalkyl radical or a hydroxyl radical, wherein 0 to 8 radicals R can be bonded to A and, in the case of more than one radical, can be identical or different.

The polythiophenes preferably in each case carry H on the end groups.

In the context of the invention, $C_1$-$C_5$-alkylene radicals A are preferably methylene, ethylene, n-propylene, n-butylene or n-pentylene. $C_1$-$C_{18}$-alkyl R preferably represent linear or branched $C_1$-$C_{18}$-alkyl radicals, such as methyl, ethyl, n- or iso-propyl, n-, iso-, sec- or tert-butyl, n-pentyl, 1-methylbutyl, 2-methylbutyl, 3-methylbutyl, 1-ethylpropyl, 1,1-dimethylpropyl, 1,2-dimethylpropyl, 2,2-dimethylpropyl, n-hexyl, n-heptyl, n-octyl, 2-ethylhexyl, n-nonyl, n-decyl, n-undecyl, n-dodecyl, n-tridecyl, n-tetradecyl, n-hexadecyl or n-octadecyl, $C_5$-$C_{12}$-cycloalkyl radicals R represent, for example, cyclopentyl, cyclohexyl, cycloheptyl, cyclooctyl, cyclononyl or cyclodecyl, $C_5$-$C_{14}$-aryl radicals R represent, for example, phenyl or naphthyl, and $C_7$-$C_{18}$-aralkyl radicals R represent, for example, benzyl, o-, m-, p-tolyl, 2,3-, 2,4-, 2,5-, 2,6-, 3,4-, 3,5-xylyl or mesityl. The preceding list serves to illustrate the invention by way of example and is not to be considered conclusive.

In the context of the invention, numerous organic groups are possible as optionally further substituents of the radicals A and/or of the radicals R, for example alkyl, cycloalkyl, aryl, aralkyl, alkoxy, halogen, ether, thioether, disulphide, sulphoxide, sulphone, sulphonate, amino, aldehyde, keto, carboxylic acid ester, carboxylic acid, carbonate, carboxylate, cyano, alkylsilane and alkoxysilane groups and carboxamide groups.

Polythiophenes in which A represents an optionally substituted $C_2$-$C_3$-alkylene radical are particularly preferred. Poly(3,4-ethylenedioxythiophene) is very particularly preferred as the polythiophene.

The polythiophenes can be neutral or cationic, wherein cationic polythiophenes are particularly preferred. The term "cationic" only relates to the charges on the polythiophene main chain. The polythiophenes can carry positive and negative charges in the structural unit, depending on the substituent on the radicals R, the positive charges being on the polythiophene main chain and the negative charges optionally being on the radicals R substituted by sulphonate or carboxylate groups. In this context, the positive charges of the polythiophene main chain can be partly or completely satisfied by the anionic groups optionally present on the radicals R. Overall, in these cases the polythiophenes can be cationic, neutral or even anionic. Nevertheless, in the context of the invention they are all regarded as cationic polythiophenes, since the positive charges on the polythiophene main chain are the deciding factor. The positive charges are

not shown in the formulae, since their precise number and position cannot be determined absolutely. However, the number of positive charges is at least 1 and at most n, where n is the total number of all recurring units (identical or different) within the polythiophene.

For compensation of the positive charge of the cationic polythiophene, it is preferred that conductive polymer further comprises a polymeric anion which is preferably based on polymers functionalized with acid groups. Anions of polymeric carboxylic acids, such as polyacrylic acids, polymethacrylic acid or polymaleic acids, or of polymeric sulphonic acids, such as polystyrenesulphonic acids and poly-vinylsulphonic acids, are possible in particular as the polyanion. These polycarboxylic and -sulphonic acids can also be copolymers of vinylcarboxylic and vinyl-sulphonic acids with other polymerizable monomers, such as acrylic acid esters and styrene. Polyanions which are furthermore possible are perfluorinated, colloid-forming polyanions, which are commercially obtainable, for example, under the name Nafion®. The molecular weight of the polymers which are functionalized with acid groups and supply the polyanions is preferably 1,000 to 2,000,000, particularly preferably 2,000 to 500,000. The polymers functionalized with acid groups or their alkali metal salts are commercially obtainable, e.g. polystyrenesulphonic acids and polyacrylic acids, or can be prepared by known processes (see e.g. Houben Weyl, Methoden der organischen Chemie, vol. E 20 Makromolekulare Stoffe, part 2, (1987), p. 1141 et seq.). A particularly preferred polyanion is an anion of polystyrene sulfonic acid.

The conductive polymer preferably is a complex of a cationic polythiophene and a polymeric anion, particularly preferably a PEDOT/PSS-complex. Such complexes are obtainable by polymerizing the thiophene monomers, preferably 3,4-ethylene-dioxythiophene, oxidatively in an aqueous solution in the presence of the polymeric anions, preferably by oxidatively polymerizing 3,4-ethylene-dioxythiophene in the presence of an anion of polystyrenesulphonic acid.

Thermoplastic polymers

The thermoplastic polymers that are preferably used for the preparation of the bodies according to the present invention in the 3D-printing process are preferably selected from the group consisting of acrylate or methylmethacrylate polymers or copolymers, acrylonitrile-butadiene-styrene polymers (ABS), polyvinylidene fluoride polymers, polyurethane polymers, polyolefin polymers, polyester polymers, polyamide polymers, polyimide polymers, styrene-ethylene-butylene styrene (SEBS) polymers, polyether ether ketone (PEEK) polymers, polyphenylene sulfide (PPS) polymers, polysulfone polymers, ethylene-vinyl acetate (EVA) polymers, poly-ethalene-naphthalate (PEN) polymers, aromatic polycarbonate polymers and mixtures of two or more of these polymers.

The term "thermoplastic polyester polymer" refers to those polyester polymers which are thermoplastic. Suitable thermoplastic polyester polymers for use herein may include one or more of the following: polylactic acid (PLA) polymers; polyhydroxyalkanoate (PHA) polymers; or polycaprolactone (PCL) polymers. These thermoplastic polyester polymers may be used in pure form or as a blend with each other, and may include other additives such as plasticizers, fillers, colorants, etc..

The term "polylactic acid or polylactide (PLA) polymers" refers to thermoplastic aliphatic polyesters formed from a lactic acid or a source of lactic acid, for example, renewable resources such as corn starch, sugarcane, etc. The term PLA may refer to all stereoisomeric forms of PLA including L- or D-lactides, and racemic mixtures comprising L- and D-lactides. For example, PLA may include D-polylactic acid, L-polylactic acid (also known as PLLA), D,L-polylactic acid, meso-polylactic acid, as well as any combination of D-polylactic acid, L-polylactic acid, D,L-polylactic acid and meso-polylactic acid. PLA polymers useful herein may have, for example, a number average molecular weight in the range of from about 15,000 to about 300,000 and may have melting points in the range of from about 150° to about 160°C. In preparing PLA polymers, bacterial fermentation may be used to produce lactic acid, which may be oligomerized and then catalytically dimerized to provide the monomer for ring-opening polymerization. PLA polymers may be prepared in a high molecular weight form through ring-opening polymerization of the monomer using, for example, a stannous octanoate catalyst, tin(II) chloride, etc. PLA polymers may be available in a variety of grades that differ in a molecular weight and degree of crystallinity Commercially available PLA polymers suitable for use herein may be obtained, for example, from Nature Works, Inc., such as the Ingeo™ 3, 4, and 6 series of PLA polymers, which includes: 3001D; 3052D; 3100HP; 325 ID; 3260HP; 4032D; 4043D; 4044D; 4060D; 6060D; 6100D; 6201D; 6202D; 6252D; 6260D; 6302D; 6361D; 6362D; 6400D; 6752D; or 10361D.

The term "polyhydroxyalkanoate (PHA) polymers" refers to thermoplastic aliphatic polyesters which may be produced by polymerization of the respective monomer hydroxy aliphatic acids (including dimers of the hydroxy aliphatic acids), by bacterial fermentation of starch, sugars, lipids, etc. PHAs may have melting points in the range of from about 40° to about 180°C and may include one or more of: poly-beta-hydroxybutyrate (PHB) (also known as poly-3-hydroxybutyrate); poly-alpha-hydroxybutyrate (also known as poly-2-hydroxybutyrate); poly-3-hydroxypropionate; poly-3-hydroxy valerate; poly-4-hydroxybutyrate; poly-4-hydroxyvalerate; poly-5-hydroxyvalerate; poly-3-hydroxyhexanoate; poly-4-hydroxyhexanoate; poly-6-hydroxyhexanoate; polyhydroxybutyrate-valerate (PHBV); etc., including copolymers, blends, mixtures, combinations, etc., of different PHA polymers, etc.. PHAs may be synthesized by methods disclosed in, for example, US 7,267,794, US 7,276,361 or US 7,208,535.

The term "polycaprolactone (PCL) polymers" refers to thermoplastic aliphatic polyesters which may be prepared by ring opening polymerization of ε-caprolactone using a catalyst such as stannous octoate and may have a melting point about 60°C.

Suitable polymers to be used in the process according to the present invention comprise Desmopan 9385 A, Vestamid X7167 nf-pA12, Conductive ABS Nanocyl, Lexan 141R, Cyrolite G20-100, Dryflex UV 902858, Medalist MD-12130H, Desmopan 9370 AU, Grilamid XE4010, Makrolon 2805, Terluran PG35, Lexan 940, Terlux 2802, Meliflex M8260BLT01, GM9450F, Adsyl 5C 30 F, FX381/17, Luvocom 3F 9936, Luvovom 3F 9875, Plexiglas 7N, Lustran ABS 348, MECO-Plast-02-001, 100-GB06, PHA 3444, Marlex HHM 5502BN, PETG-T, PBS, PCU Bionate55D and mixtures thereof.

The process according to the present invention

The process for the preparation of the bodies according to the present disclosure is preferably a 3D-printing process, wherein the body is prepared in layers by depositing droplets of a molten polymer onto the surface of a substrate or - if the layer is not the very first layer - onto the surface of a previously applied layer, wherein in the preparation of at least some of the layers a first area and a second area are formed which preferably differ with respect to the density in which the droplets are deposited.

Such a process can be performed using devices such as the Arburg Freeformer (Arburg GmbH + Co. KG, Loβburg, Germany). This is a device for the production of a three-dimensional objects made of a solidifiable material (which is either present in a fluid phase in its initial state or can be fluidified) by a sequential discharge of drops as disclosed in US 2012/156319 A1. The device comprises

- at least one construction space for constructing the object,
- at least one processing unit for processing of the solidifiable material into the fluid phase,
- at least one material reservoir for the fluid phase with at least one discharge unit for the sequential discharge of the solidifiable material in the form of discrete drops in direction of the construction space through a discharge orifice furnished with a synchronizable closing mechanism,
- at least one pressure generating unit for generating pressure on the fluid phase in the material reservoir,

wherein the closing mechanism has an elastic deformable solid body joint.

The Arburg Freeformer is characterized by a piezo nozzle cap that opens and closes up to a hundred times per second to produce tiny droplets of thermoplastic material. The Freeformer builds up the three-dimensional object layer by layer from the droplets. The desired volume of the drop preferably lies in the range from 0.01 to 0.5 $mm^3$, preferably in the range of 0.05 to 0.3 $mm^3$ and in particular preferably in the region of about 0.1 $mm^3$. The diameter of the discharge opening is preferably smaller or equal to 1 mm, more preferably about 0.1 mm. The layer thickness results from the respective droplet height and usually varies between 0.14 and 0.34 mm, also depending on the nozzle that is used. The shape of the discharged droplets is also influenced by the viscosity of the material.

Printers such as the Arburg Freeformer 200-3X or 300-3X not only allow the use of two or more different thermoplastic materials for the formation of a three-dimensional object, they are also able to deposit these different materials with different filling factors when depositing the droplets of the molten materials. It is thus possible to prepare three-dimensional objects having areas with a different porosity (and which additionally may comprise polymers with a different compression modulus) which, depending on their porosity, may be selectively impregnated with conductive materials in order to increase the conductivity in certain areas or the three-dimensional object in a targeted way.

In a 3D-printing process that is based on additive layer manufacturing (ALM), such as the printing process that is performed when using the Arburg Freeformer, process, the 3D-body is usually formed layer-up-layer, each layer being formed line-next to-line in a given layer and each line being formed droplet-next to droplet in a given line. To adjust the porosity of a given segment in a 3D-printing process by means of 3D-printers such as the Arburg Freeformer

i) the distance in which two subsequent droplets in a given line are deposited next to each other onto a substrate or onto a layer that has been previously printed can be varied (for example by adjusting the frequency in which piezo nozzle cap in an Arburg Freeformer opens and closes),

ii) the distance in which two consecutive print lines are printed in a given layer can be varied (by an appropriate positioning of the printing nozzle relative to the substrate onto which the drops are to be deposited), or

iii) measures i) and ii) can be combined.

Furthermore, when forming a given layer of a 3D-object by means of the above described additive layer manufacturing (ALM) process it may also be advantageous to additional print a supporting polymer in the outer area of each layer (which in the finished 3D-object than covers the outer surface of the body or at least a part of it) in order to provide a sufficient stability of the 3D-object and to improve the form stability during the printing process. In this context thermoplastic polymers such as Armat 21 (Arburg, Germany) are preferred which later can easily be removed by means of alkaline aqueous solutions.

According to a first preferred embodiment of the process according to the present invention only one thermoplastic material is used for the formation of both, the first and the further segment, wherein for the formation of the first and the further segment this thermoplastic material is deposited in different filling factors, which in turn leads to a different porosity of the first and the further segment. To obtain the desired difference in the electrical conductivity of the first and the further segment, the first segment is - after the formation of the body - brought into contact with a conductive material. This can be accomplished by selectively coating the outer surfaces of the first segment with a conductive material, by impregnating the first segment with a conductive material (such as a PEDOT/PSS-dispersion) or by a combination of these approaches. The thermoplastic material that is used for the formation of the first segment can be a non-conductive thermoplastic polymer or a conductive material that comprises a non-conductive thermoplastic polymer and a conductive component.

According to a second preferred embodiment of the process according to the present invention two different thermoplastic materials are used for the formation of the first and the further segment, wherein for the formation of the first and the further segment these thermoplastic materials are also deposited in different filling factors, which in turn leads to a first and a further segment which differ in both, the material from which they have been formed and the porosity. Thus, for the formation of the first polymer segment a first thermoplastic material can be used (which may be a conductive material having a first conductivity or a non-conductive material) and for the formation of the further segment a second thermoplastic material can be used. If the two materials differ with respect to their electrical conductivity, the process directly leads to the formation of a body comprising two segments which differ with respect to both, their porosity and their electrical conductivity. If two non-conductive materials are used, the conductivity in the first segment can than be further improved by one of the approaches described before (i. e. by coating the outer surface of first segment with a further conductive material or by impregnating the first segment with a conductive material).

For the formation of the first and the further segment in the process according to the present invention, particularly in the process according to the first and the second embodiment as described above,

- the first segment is prepared using a fill factor f1 in the range from 5 to 100 %, preferably in the range from 10 to 90 %, more preferably in the range from 15 to 80 % and even more preferably in the range from 25 to 70 %;

- the further segment is prepared using a fill factor f1 in the range from 5 to 100 %, preferably in the range from 10 to 90 %, more preferably in the range from 15 to 80 % and even more preferably in the range from 20 to 70 %.

Furthermore, it is also preferred that for the formation of the first and the further segment in the process according to the present invention, particularly in the process according to the first and the second embodiment as described above, the following condition is fulfilled:

$$f2 > 1.25 \times f1,$$

more preferably $f2 > 1.5 \times f1$,
even more preferably $f2 > 1.75 \times f1$,
even more preferably $f2 > 2 \times f1$,
even more preferably $f2 > 4 \times f1$.

In an aspect, the disclosure provides a process for the preparation of a body (100), comprising

I) preparing, by means of 3D-printing, at least one first polymer segment (101) having a porosity p1, preferably by using a first fill factor f1;
II) preparing, by means of 3D-printing, at least one further polymer segment (102) being in seamless contact with the at least one first polymer segment (101);
III) optionally bringing into contact a conductive material with a segment selected from the first polymer segment (101) and the further polymer segment (102);

characterized in that the least one further polymer segment (102) has a porosity $p2 \neq p1$, preferably by using a second fill factor f2 # f1.

The difference in the porosity between the first and the further polymer segment can not only be controlled by adjusting the fill factor, but also by other measures such as the use of blowing agents.

In an aspect, the body (100) is prepared in layers (109) by depositing droplets (104) of a molten polymer onto a surface of a substrate (105) or onto a surface of a previously applied layer (109), preferably onto the outer surface of a previously applied layer (109), wherein in the preparation of at least some of the layers (109) a first area (106) and a second area (107) are formed which differ with respect to the density in which the droplets (104) are deposited. The first and the second area in the two-dimensional form result in the first and the second segment in the three-dimensional form of the body if the individual layers are printed on top of each other in the 3D printing process.

In an aspect, at least one segment selected from the first polymer segment (101) and the further polymer segment (102) is made from a conductive material that comprises a non-conductive polymer matrix and a conductive component dispersed therein or at least one segment selected from the first polymer segment (101) and the further polymer segment (102) is made from a non-conductive material.

In an aspect, at least one segment selected from the first polymer segment (101) and the further polymer segment (102) comprises void spaces (108) and wherein in process step III) a conductive component is introduced into at least some of the void spaces (108) or wherein in process step III) at least a part of the surface of at least one segment selected from the first polymer segment (101) and the further polymer segment (102) is coated with a conductive component.

As an aspect, the disclosure also provides a body 2, comprising

i) at least one first polymer segment (101) having a porosity p1 and an electrical conductivity c1, and
ii) at least one further polymer segment (102) being in seamless contact with the at least one first polymer segment (101) and having a porosity $p2 \neq p1$ and an electrical conductivity c2 # c1,

**characterized in that** the first polymer segment (101) has an electrical conductivity c1 of at least 0.001 S/cm and wherein the further polymer segment (102) has an electrical conductivity c2 of less than $1 \times 10^{-9}$ S/cm.

In an aspect, $p1 > p2$.

In an aspect, at least one segment selected from the first polymer segment (101) and the further polymer segment (102) is made from a conductive material that comprises a non-conductive polymer matrix and a conductive component dispersed therein.

In an aspect, the at least one first polymer segment (101) has a compression modulus m1 and wherein the further polymer segment has a compression modulus of $m2 \neq m1$, wherein the first polymer segment has a compression modulus m1 of less than 5000 MPa and wherein the at least one further polymer segment has a compression modulus m2 of at least 0.01 MPa.

Preferably, the conductive component comprises a conductive polymer. Preferably, the conductive polymer comprises polythiophene. Preferably, the polythiophene is a cationic polythiophene that is present in the form of a polythiophene/polyanion-complex. Preferably, the polythiophene/polyanion-complex is a PEDOT/PSS-complex.

In an aspect, the further polymer segment (102) is made from a non-conductive material.

In an aspect, the body (100) is comprised in a device selected from the group consisting of, or is a device selected from the group consisting of, a biosensor, a body-worn device having electrically sensing functionality and a body-worn device selected from the group consisting of a headphone, glasses, a headband, a chest-strap, an armband, a patch, a garment or a wrist-worn device.

TEST METHODS

The following test methods are used in the invention. Any examples falling outside the scope of the claims are provided for comparative purposes only.

In absence of a test method, the ISO test method for the feature to be measured being closest to the earliest filing date of the present application applies. In absence of distinct measuring conditions, standard ambient temperature and pressure (SATP) as a temperature of 298.15 K (25 °C, 77 °F) and an absolute pressure of 100 kPa (14.504 psi, 0.986 atm) apply.

To determine the porosity, the electrical conductivity and the compression modulus in the different segments of the body according to the present invention the test methods described below can be used. If the size of the segments is to small or if the segments in the body are not accessible to determine these parameters, it is recommended to prepare these segments in individual form and in sizes that are required for the specimens used in these test methods, wherein specimens of the segments are printed by 3D-printing under the same conditions (fill factor, temperature and viscosity of the molten polymer etc.) as they have been printed when preparing the body according to the present invention or when applying the process according to the present invention.

Porosity

The porosity of can determined one the basis of the geometrical density with the following steps:

1) Dry the sample for at least 2h at 85 °C.

2) Measure the exact geometry of the sample, preferably contact less as with a Mitutoyo S200Z, and calculate the volume V.

3) Measure the mass m of the sample.

4) Calculate the bulk density $\rho_B = m/V$.

5) Calculate porosity P in % with the theoretical density $\rho_{theo}$ of the material as

$$P = (\rho_{theo} - \rho_B)/\ \rho_{theo}\ 100\%.$$

Electrical conductivity

The electrical conductivity is determined according to ASTM B193 (Standard Test Method for Resistivity of Electrical Conductor Materials) with the following steps:

1) A bar of 30 mm length l of the sample is provided having a uniform square cross-section *A* of 5 mm edge length.

2) The dimensions are determined using contact-less measurement such as measurement projector Mitutoyo QS200Z.

3) The resistance *R* is measured.

4) The conductivity is calculated as $\sigma = 1/p = 1/(A \times R)$.

Compression modulus

The compression modulus of the first and the further segment of the body according to the present invention can be determined on the basis of DIN EN ISO 3386-2, with the following steps:

1) Three samples of each segment were measured whereas each sample is a cylinder with a thickness of 15 mm and a diameter of 5 mm whereas the z-direction of the sample (that is, along the thickness) along which the force will be applied coincides with the z-direction during printing.

2) Measurement is conducted at least 72h after production of the samples and after at least 24 h of conditioning the undeflected, undistorted samples at 23°C at 50% relative humidity.

3) Compression is performed with 100 mm/min until a compression of 70% of the initial test piece thickness is achieved and then decompression to the initial test piece thickness is performed.

4) The procedure is repeated immediately three times while the fourth compression cycle is recorded for evaluation.

5) The slope of the initial linear section in the compression-strain curve is the Young's modulus Y from which the compression modulus K is calculated as

$$K = Y / (3\text{-}6\nu) \text{ with the Poisson ratio } \nu \text{ approximated with } 0.3.$$

EXAMPLES

The present invention is now explained in more detail by examples and drawings given by way of example

which do not limit it. Any examples falling outside the scope of the claims are provided for comparative purposes only.

**Example 1**

A three-dimensional body as shown in figure 2A that can later be used as an in-ear multiple electrode has been printed in an Arburg Freeformer 300-3X using the following conditions and parameters:

| | mass preparation 1 | masspreparation 2 | mass preparation 3 | unit |
|---|---|---|---|---|
| material | ABS (Terluran GP35 300-3X) | Armat 21 300-3X | Thermoflex EC 80.1 | |
| nozzle diameter (built in) | 0.2 | 0.2 | 0.2 | mm |
| layer thickness | 0.2 | | | mm |
| number of layers | 124 | | | |
| droplet closing factor | 367 | 487 | 565 | $\times 10^3$ |
| number of droplets | 100 | 105 | 565 | $\times 10^3$ |
| material feed temperature | 35 | 45 | 35 | °C |
| temperature zone 1 | 190 | 210 | 160 | °C |
| temperature zone 2 | 220 | 235 | 190 | °C |
| temperature deposition zone | 240 | 220 | 210 | °C |
| temperature construction space | 90 | | | °C |
| lowering temperature zone 1 | 100 | 100 | 100 | °C |
| lowering temperature construction zone | 90 | | | °C |
| melt cushion | 1.5 | 1.5 | 1.5 | mm |
| circumferential speed | 4 | 4 | 3 | m/min |
| dynamic pressure | 50 | 40 | 40 | bar |
| metering stroke | 10 | 10 | 6 | mm |

The ABS-polymer forms the "further segment" of the body according to the present invention and constituted the matrix polymer into which a conductive segment that is formed from the PTS-polymer (i. e. the "first segment" of the body according to the present invention) is embedded and on the surface of which such a conductive segment is localized.

The figures show

1) a cross-sectional scheme of the body according to the invention;
2A) a side view of the body according to the invention in the design of an in-ear multiple electrode;
2B) the in-ear multiple electrode shown in figure 2A) in a top view;
3) a scheme showing the deposition of droplets onto the surface of a substrate in a 3D-printing process according to the invention;
4) two further schemes showing the deposition of droplets onto the surface of a substrate in a 3D-printing process according to the invention, in which three successive sheet layers are shown;
5) the formation of the body shown in figure 1 by means of the process according to the invention;
6) a scheme showing the process step of impregnating certain segments of the body according to the invention shown in figure 1 with PEDOT/PSS;
7) the formation of the body shown in figure 2a by means of the process according to the invention;
8A) a scheme showing the process step of introducing PEDOT in certain segments of the body according to the invention shown in figure 2A;
8B) a further scheme showing the process step of introducing PEDOT in certain segments of the body according to the invention shown in figure 2A.

Figure 1A) is a cross-sectional scheme of the body 100 according to the invention, wherein the body 100 comprises at least one first segment 101 and at least one further segment 102, wherein these segments differ in both, the porosity and the electrical conductivity. The first and the further segment 101,102 are in seamless contact with each other. When producing the body 100 according to the present invention by means of a 3D-printing in the border area between these two segments droplets 104 (see figures 3 and 4) of the molten thermoplastic material used to prepare the first segment 101 and droplets 104 (see figures 3 and 4) of the molten thermoplastic material used to prepare the further segment 102 flow into each (i. e. they form a coherent material upon solidification of the molten thermoplastic material).

Figure 2A is a cross-sectional scheme of the body 100 according to the invention in the design of an in-ear multiple electrode 103. Figure 2B shows the same the in-ear multiple electrode 103 in a top view. The multiple electrode 103 comprises first segments 101 forming the electrodes and a further segment 102 serving as the base in which the electrodes 101 are embedded (see the pathways 101 on the left that are shaded in dark grey) or onto they have been deposited (see the pathways 101 on the top and on the right that are shaded in black and that preferably serve for direct skin contact). As shown in Figure 2A, the embedded pathway 101 contacts the electrode 101 on the top of the multiple electrode 103 and thus allows a connection of that electrode 101 with a measuring device (not shown in figure 2A). Preferably, in such an in-ear multiple electrode 103 the base 102 is made from a soft and deformable material, whereas the electrodes are manufactured from a harder material. When producing the in-ear multiple electrode 103 by means of a 3D-printing process the thermoplastic material used to prepare the base 102 is preferably a non-conductive thermoplastic material, which upon solidification forms a soft material, whereas the electrodes 101 are either formed from a conductive thermoplastic material or from a non-conductive thermoplastic material which, after the formation of the multiple electrode 103 is brought into contact with a conductive material in order to provide the required conductivity (as also shown in figure 8).

Figure 3 is a scheme illustrating the deposition of droplets 104 onto the surface of a substrate 105 in a 3D-printing process according to the invention. As shown in figure 3 two different areas 106 and 107 are formed in which the droplets are deposited in a different density. In a three-dimensional structure in which by means of such as process several successive layers 109, 109a, 109b, 109c are produced (see figures 4, 5 and 7), polymer bodies 100 are obtained comprising two or more different segments 101, 102 which differ with respect to their porosity. If, in addition to the different density in which the droplets 104 are deposited, also different thermoplastic materials are used, bodies 100 can be obtained in which the segments 101, 102 not only differ with respect to their porosity, but also with respect to their compression modulus (i. e. their softness). In order to obtain the desired difference in the electrical conductivity of the segments 101, 102 it is possible to either use thermoplastic materials which differ with respect to their electrical conductivity and/or to bring one of these segments into contact with a conductive material (or to combined these measures). Preferably, the segment with the higher porosity is impregnated with a conductive material such as a conductive polymer or a conductive polymer is formed within the pores 108 of the segment with the higher porosity.

Figure 4 shows two further schemes illustrating the deposition of droplets 109 in a 3D-printing process according to the invention, in which three successive sheet layers 109a, 109b and 109c are shown. As can be seen from that figure, pores 108 are formed the size of which can be adjusted by the printing density that is used. In a subsequent process step a conductive material can be introduced into these pores 108 in order to increase the electrical conductivity in selected areas of the body 100.

Figure 5 illustrates the formation of the body 100 shown in figure 1 by means of the process according to the invention. As can be seen, layers 109 which comprise areas 106 (grey), 107 (black) in which the droplets of the thermoplastic polymer have been deposited in a different density (as shown in figures 3 and 4) are formed one after another, thereby obtaining the polymer body with a structure as shown in figure 1 (not all the layers that are necessary to form the electrode of figure 1 are shown). The first polymer segment 101 that is obtained by the successive deposition of the areas 107 is characterized by a higher porosity, compared to the second polymer segment 102 that is obtained by the successive deposition of the areas 106.

Figure 6 is a scheme showing the process step of impregnating certain segments of the body according to the invention shown in figure 1 with PEDOT/PSS. The body 100 in the centre of figure 6 corresponds to the one shown in figure 1 and obtained by the process shown in figure 5. The body on the left is not according to the present invention whereas the body 100 on the right is a slight modification of the body 100 in the centre. The bodies 100 in the centre and on the right comprise a first segment 101 in the outer region that is characterized by a high porosity and a further region 102 serving as the base of the body 100. To provide the more porous segment 101 with the desired electrical conductivity, the bodies are impregnated with a conductive material, such as a PEDOT/PSS-dispersion. Suitable dispersions that can be used for that purpose are formulation such as Tecticoat™ (Heraeus, Germany). In the alternative, it is also possible to impregnate the more porous regions with compositions comprising monomers of a conductive polymer and to polymerize these monomers *in situ* for the formation of a conductive polymer by means of electrodeposition. Compositions suitable for that purpose are formulation such as Amplicoat™ (Heraeus, Germany). Furthermore, the more porous region can also be impregnated with conductive inks based on metal particles or metal-organic complexes. To limit the impregnation of the more porous regions with conductive materials to well defined areas, it is also advantageous to apply a protective coating 111 onto those areas that are not intended to be impregnated (as shown in figure 8A).

Figure 7 shows the formation of the in-ear multiple electrode 103 that is illustrated in figure 2A by means of the 3D-printing process according to the invention. As can be seen, the in-ear multiple electrode 103 is formed layer-by-layer (again not all the layers that are necessary to form such an electrode are shown), wherein at least some of the layers 109 comprise areas 106,107 in which the drops 104 of the thermoplastic material have been deposited in a different density (as shown in figures 3 and 4). The process shown in figure 7 leads to a body 100 with the structure of the in-ear multiple electrode shown in figure 2A which comprises a first segment 101 serving for the formation of the electrodes and a further segment 102 serving as the base in which the electrodes 101 are embedded.

Figures 8A and 8B show a scheme illustrating the process step of introducing PEDOT in certain segments of the in-ear multiple electrode 103 that has been obtained by the process shown in figure 7. According to the approach shown in figure 8a the outer surface of the multiple electrode 103 is partially covered with a polymer coating 110. The areas of the first segment 101, however, remain uncoated. In order to increase the electrical conductivity in the first segment 101, PEDOT/PSS, for example in form of the formulation Tecticoat™, is sprayed upon these surfaces. Upon drying and removal of the protective polymer coating 111 an in-ear multiple electrode 103 as shown in figure 2a is obtained in which the PEDOT/PSS-impregnated areas form the electrodes. According to the approach shown in figure 8b the multiple electrode 103 that has been obtained by the process shown in figure 7 is impregnated with a composition comprising monomers of a conductive polymer and the monomers are subsequently polymerized *in situ* for the formation of a conductive polymer by means of electrodeposition. Compositions suitable for that purpose are formulation such as Amplicoat™ (Heraeus, Germany). As can be seen in figure 8B, this process is particularly suitable to also increase the electrical conductivity in segments of the body 100 which are completely embedded in the base (see segment with reference number 102 in figure 8B).

LIST OF REFERENCES

| | |
|---|---|
| **100** | body according to the invention |
| **101** | first segment |
| **102** | further segment |
| **103** | body according to the invention in the design of an in-ear multiple electrode |
| **104** | droplet |
| **105** | substrate |
| **106** | first area |
| **107** | second area |
| **108** | voids |
| **109** | sheet polymer coating |
| **109a** | first sheet |
| **109b** | second sheet |
| **109c** | third sheet |
| **110** | supporting polymer |
| **111** | protective polymer coating |

## Claims

1. A body (100), comprising

i) at least one first polymer segment (101) having a porosity p1 and an electrical conductivity c1, and
ii) at least one further polymer segment (102) being in seamless contact with the at least one first polymer segment (101) and having a porosity p2 ≠ p1 and an electrical conductivity c2 # c1,

**characterized in that** the first polymer segment (101) has an electrical conductivity c1 of at least 0.001 S/cm and wherein the further polymer segment (102) has an electrical conductivity c2 of less than $1 \times 10^{-9}$ S/cm.

2. The body (100) according to claim 1, wherein

$$p1 > p2.$$

3. The body (100) according to claim 1 or 2, wherein at least one segment selected from the first polymer segment (101) and the further polymer segment (102) is made from a conductive material that comprises a non-conductive polymer matrix and a conductive component dispersed therein.

4. The body (100) according to claim 1, wherein the at least one first polymer segment (101) has a compression modulus m1 and wherein the further polymer segment has a compression modulus of m2 ≠ m1, wherein the first polymer segment has a compression modulus m1 of less than 5000 MPa and wherein the at least one further polymer segment has a compression modulus m2 of at least 0.01 MPa.

5. The body (100) according to claim 4, wherein the conductive component comprises a conductive polymer.

6. The body (100) according to claim 5, wherein the conductive polymer comprises a polythiophene.

7. The body (100) according to claim 6, wherein the polythiophene is a cationic polythiophene that is present in the form of a polythiophene/polyanion-complex.

8. The body (100) according to claim 7, wherein the polythiophene/polyanion-complex is a PEDOT/PSS-complex.

9. The body (100) according to anyone of claims 1 to 4, wherein the further polymer segment (102) is made from a non-conductive material.

10. The body (100) according to anyone of claims 1 to 9, wherein the body (100) is comprised in a device selected from the group consisting of, or is a device selected from the group consisting of, a biosensor, a body-worn device having electrically sensing functionality and a body-worn device selected from the group consisting of a headphone, glasses, a headband, a chest-strap, an armband, a patch, a garment or a wrist-worn device.

11. A process for the preparation of a body (100), comprising

   I) preparing, by means of 3D-printing, at least one first polymer segment (101) having a porosity p1, preferably by using a first fill factor f1;
   II) preparing, by means of 3D-printing, at least one further polymer segment (102) being in seamless contact with the at least one first polymer segment (101);
   III) bringing into contact a conductive material with a segment selected from the first polymer segment (101) and the further polymer segment (102);

   **characterized in that** the least one further polymer segment (102) has a porosity p2 ≠ p1, preferably by using a second fill factor f2 # f1.

12. The process according to claim 11, wherein the body (100) is prepared in layers (109) by depositing droplets (104) of a molten polymer onto a surface of a substrate (105) or onto a surface of a previously applied layer (109), preferably onto the outer surface of a previously applied layer (109), wherein in the preparation of at least some of the layers (109) a first area (106) and a second area (107) are formed which differ with respect to the density in which the droplets (104) are deposited.

13. The process according to claim 11 or 12, wherein at least one segment selected from the first polymer segment (101) and the further polymer segment (102) is made from a conductive material that comprises a non-conductive polymer matrix and a conductive component dispersed therein.

14. The process according to claim 11 or 12, wherein at least one segment selected from the first polymer segment (101) and the further polymer segment (102) is made from a non-conductive material.

15. The process according to anyone of claims 11 to 14, wherein at least one segment selected from the first polymer segment (101) and the further polymer segment (102) comprises void spaces (108) and wherein in process step III) a conductive component is introduced into at least some of the void spaces (108) or wherein in process step III) at least a part of the surface of at least one segment selected from the first polymer segment (101) and the further

polymer segment (102) is coated with a conductive component.

## Patentansprüche

**1.** Körper (100), umfassend

i) mindestens ein erstes Polymersegment (101) mit einer Porosität p1 und einer elektrischen Leitfähigkeit c1, und
ii) mindestens ein weiteres Polymersegment (102), das in lückenlosem Kontakt mit dem mindestens einen ersten Polymersegment (101) ist und eine Porosität p2 # p1 und eine elektrische Leitfähigkeit c2 # c1 aufweist,

**dadurch gekennzeichnet, dass** das erste Polymersegment (101) eine elektrische Leitfähigkeit c1 von mindestens 0,001 S/cm aufweist und wobei das weitere Polymersegment (102) eine elektrische Leitfähigkeit c2 von weniger als $1 \times 10^{-9}$ S/cm aufweist.

**2.** Körper (100) nach Anspruch 1, wobei

$$p1 > p2.$$

**3.** Körper (100) nach Anspruch 1 oder 2, wobei mindestens ein Segment, ausgewählt aus dem ersten Polymersegment (101) und dem weiteren Polymersegment (102), aus einem leitenden Material besteht, das eine nichtleitende Polymermatrix und eine darin dispergierte leitende Komponente umfasst.

**4.** Körper (100) nach Anspruch 1, wobei das mindestens eine erste Polymersegment (101) einen Kompressionsmodul m1 aufweist und wobei das weitere Polymersegment einen Kompressionsmodul von m2 # m1 aufweist, wobei das erste Polymersegment einen Kompressionsmodul m1 von weniger als 5000 MPa aufweist und wobei das mindestens eine weitere Polymersegment einen Kompressionsmodul m2 von mindestens 0,01 MPa aufweist.

**5.** Körper (100) nach Anspruch 4, wobei die leitende Komponente ein leitendes Polymer umfasst.

**6.** Körper (100) nach Anspruch 5, wobei das leitende Polymer ein Polythiophen umfasst.

**7.** Körper (100) nach Anspruch 6, wobei das Polythiophen ein kationisches Polythiophen ist, das in Form eines Polythiophen/Polyanion-Komplexes vorliegt.

**8.** Körper (100) nach Anspruch 7, wobei der Polythiophen/Polyanion-Komplex ein PEDOT/PSS-Komplex ist.

**9.** Körper (100) nach einem der Ansprüche 1 bis 4, wobei das weitere Polymersegment (102) aus einem nichtleitenden Material besteht.

**10.** Körper (100) nach einem der Ansprüche 1 bis 9, wobei der Körper (100) umfasst ist in einer Vorrichtung ausgewählt aus der Gruppe bestehend aus oder eine Vorrichtung ist ausgewählt aus der Gruppe bestehend aus einem Biosensor, einer am Körper getragenen Vorrichtung mit elektrischer Sensorfunktionalität und einer am Körper getragenen Vorrichtung ausgewählt aus der Gruppe bestehend aus einem Kopfhörer, einer Brille, einem Kopfband, einem Brustgurt, einem Armband, einem Patch, einem Kleidungsstück oder einer am Handgelenk getragenen Vorrichtung.

**11.** Verfahren zur Herstellung eines Körpers (100), umfassend

I) Herstellen, mittels 3D-Drucken, mindestens eines ersten Polymersegments (101) mit einer Porosität p1, vorzugsweise durch Verwendung eines ersten Füllfaktors f1;
II) Herstellen, mittels 3D-Drucken, mindestens eines weiteren Polymersegments (102), das in lückenlosem Kontakt mit dem mindestens einen ersten Polymersegment (101) ist;
III) Inkontaktbringen eines leitenden Materials mit einem Segment ausgewählt aus dem ersten Polymersegment (101) und dem weiteren Polymersegment (102);

**dadurch gekennzeichnet, dass** das mindestens eine weitere Polymersegment (102) eine Porosität p2 # p1 aufweist, vorzugsweise durch Verwendung eines zweiten Füllfaktors f2 # f1.

**12.** Verfahren nach Anspruch 11, wobei der Körper (100) in Schichten (109) hergestellt wird, indem Tröpfchen (104) eines geschmolzenen Polymers auf eine Oberfläche eines Substrats (105) oder auf eine Oberfläche einer zuvor aufgetragenen Schicht (109), vorzugsweise auf die Außenfläche einer zuvor aufgetragenen Schicht (109), aufgetragen werden, wobei bei der Herstellung mindestens einiger der Schichten (109) ein erster Bereich (106) und ein zweiter Bereich (107) gebildet werden, die sich hinsichtlich der Dichte, in der die Tröpfchen (104) abgeschieden werden, unterscheiden.

**13.** Verfahren nach Anspruch 11 oder 12, wobei mindestens ein Segment, ausgewählt aus dem ersten Polymersegment (101) und dem weiteren Polymersegment (102), aus einem leitenden Material hergestellt ist, das eine nichtleitende Polymermatrix und eine darin dispergierte leitende Komponente umfasst.

**14.** Verfahren nach Anspruch 11 oder 12, wobei mindestens ein Segment, ausgewählt aus dem ersten Polymersegment (101) und dem weiteren Polymersegment (102), aus einem nichtleitenden Material besteht.

**15.** Verfahren nach einem der Ansprüche 11 bis 14, wobei mindestens ein Segment, ausgewählt aus dem ersten Polymersegment (101) und dem weiteren Polymersegment (102), Hohlräume (108) umfasst und wobei in Verfahrensschritt III) eine leitende Komponente in mindestens einige der Hohlräume (108) eingebracht wird oder wobei in Verfahrensschritt III) mindestens ein Teil der Oberfläche mindestens eines Segments, ausgewählt aus dem ersten Polymersegment (101) und dem weiteren Polymersegment (102), mit einer leitenden Komponente beschichtet wird.

**Revendications**

**1.** Corps (100), comprenant :

i) au moins un premier segment polymère (101) ayant une porosité p1 et une conductivité électrique c1, et
ii) au moins un autre segment polymère (102) en contact sans soudure avec l'au moins un premier segment polymère (101) et ayant une porosité $p2 \neq p1$ et une conductivité électrique $c2 \neq c1$,

le corps étant **caractérisé en ce que** le premier segment polymère (101) a une conductivité électrique c1 d'au moins 0,001 S/cm et **en ce que** l'autre segment polymère (102) a une conductivité électrique c2 inférieure à $1 \times 10^{-9}$ S/cm.

**2.** Corps (100) selon la revendication 1, dans lequel :

$$p1 > p2.$$

**3.** Corps (100) selon la revendication 1 ou 2, dans lequel au moins un segment choisi parmi le premier segment polymère (101) et l'autre segment polymère (102) est constitué d'un matériau conducteur qui comprend une matrice polymère non conductrice et un composant conducteur dispersé dans celle-ci.

**4.** Corps (100) selon la revendication 1, dans lequel l'au moins un premier segment polymère (101) a un module de compression m1 et l'autre segment polymère a un module de compression $m2 \neq m1$, le premier segment polymère ayant un module de compression m1 inférieur à 5000 MPa et l'au moins un autre segment polymère ayant un module de compression m2 d'au moins 0,01 MPa.

**5.** Corps (100) selon la revendication 4, dans lequel le composant conducteur comprend un polymère conducteur.

**6.** Corps (100) selon la revendication 5, dans lequel le polymère conducteur comprend un polythiophène.

**7.** Corps (100) selon la revendication 6, dans lequel le polythiophène est un polythiophène cationique présent sous la forme d'un complexe polythiophène/polyanion.

**8.** Corps (100) selon la revendication 7, dans lequel le complexe polythiophène/polyanion est un complexe PEDOT/PSS.

**9.** Corps (100) selon l'une quelconque des revendications 1 à 4, dans lequel l'autre segment polymère (102) est

constitué d'un matériau non conducteur.

**10.** Corps (100) selon l'une quelconque des revendications 1 à 9, dans lequel le corps (100) est compris dans un dispositif choisi ou est un dispositif choisi dans le groupe constitué d'un biocapteur, d'un dispositif mettable ayant une fonctionnalité de détection électrique et d'un dispositif mettable choisi dans le groupe constitué d'un casque, de lunettes, d'un bandeau, d'une sangle de poitrine, d'un brassard, d'un patch, d'un vêtement ou d'un dispositif porté au poignet.

**11.** Procédé de préparation d'un corps (100), consistant à :

I) préparer, au moyen d'une impression 3D, au moins un premier segment polymère (101) ayant une porosité p1, de préférence au moyen d'un premier facteur de remplissage f1 ;
II) préparer, au moyen d'une impression 3D, au moins un autre segment polymère (102) en contact sans soudure avec l'au moins un premier segment polymère (101) ;
III) mettre en contact un matériau conducteur avec un segment choisi parmi le premier segment polymère (101) et l'autre segment polymère (102) ;

le procédé étant **caractérisé en ce que** l'au moins un autre segment polymère (102) a une porosité p2 ≠ p1, de préférence au moyen d'un second facteur de remplissage f2 ≠ f1.

**12.** Procédé selon la revendication 11, dans lequel le corps (100) est préparé en couches (109) en déposant des gouttelettes (104) d'un polymère fondu sur une surface d'un substrat (105) ou sur une surface d'une couche (109) appliquée précédemment, de préférence sur la surface extérieure d'une couche (109) appliquée précédemment, et dans lequel sont formées, lors de la préparation d'au moins certaines des couches (109), une première zone (106) et une seconde zone (107) qui diffèrent en ce qui concerne la densité de dépôt des gouttelettes (104).

**13.** Procédé selon la revendication 11 ou 12, dans lequel au moins un segment choisi parmi le premier segment polymère (101) et l'autre segment polymère (102) est constitué d'un matériau conducteur qui comprend une matrice polymère non conductrice et un composant conducteur dispersé dans celle-ci.

**14.** Procédé selon la revendication 11 ou 12, dans lequel au moins un segment choisi parmi le premier segment polymère (101) et l'autre segment polymère (102) est constitué d'un matériau non conducteur.

**15.** Procédé selon l'une quelconque des revendications 11 à 14, dans lequel au moins un segment choisi parmi le premier segment polymère (101) et l'autre segment polymère (102) comprend des espaces vides (108) et dans lequel, à l'étape III) du procédé, un composant conducteur est introduit dans au moins certains des espaces vides (108) ou dans lequel, à l'étape III) du procédé, au moins une partie de la surface d'au moins un segment choisi parmi le premier segment polymère (101) et l'autre segment polymère (102) est revêtue d'un composant conducteur.

Fig. 1

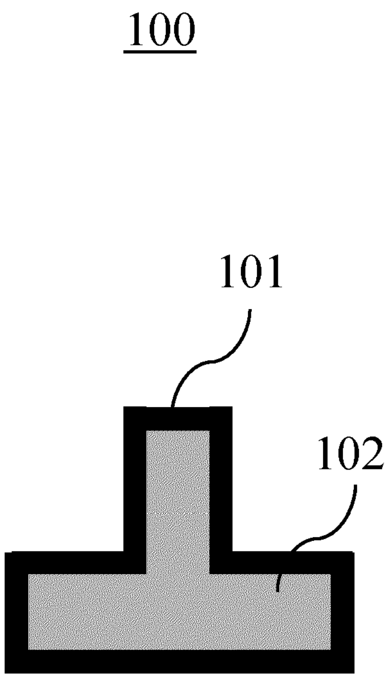

Fig. 2

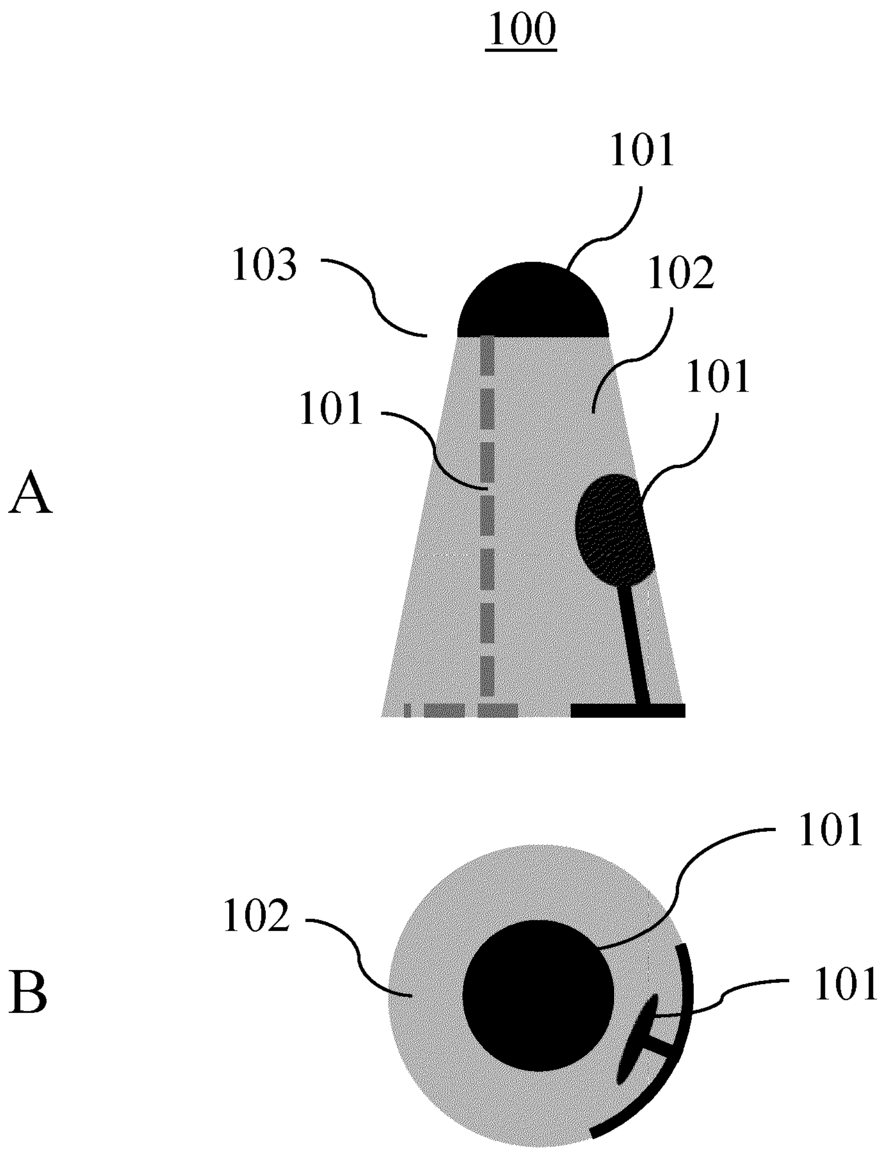

Fig. 3

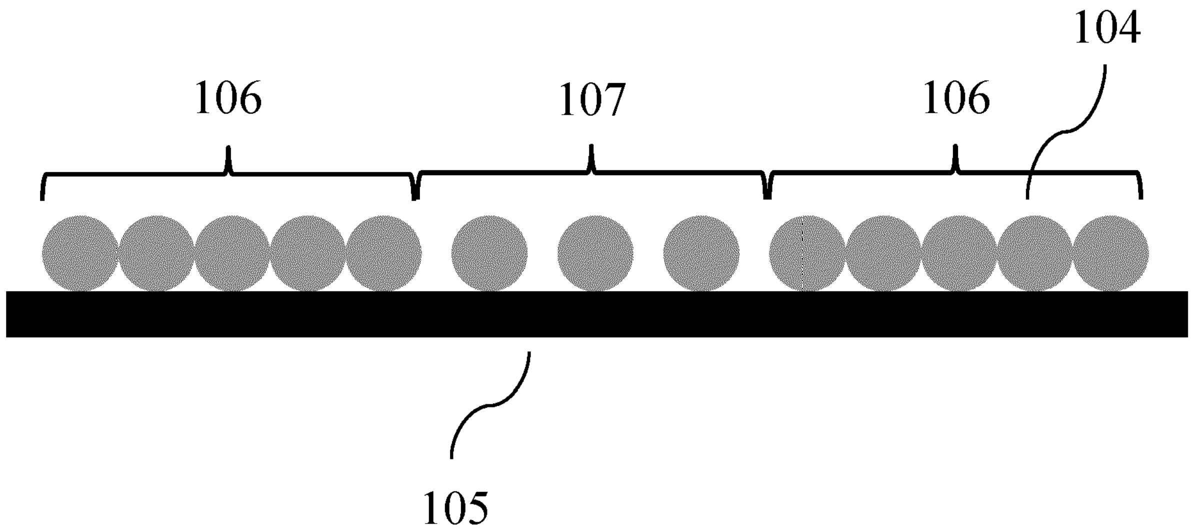

Fig. 4

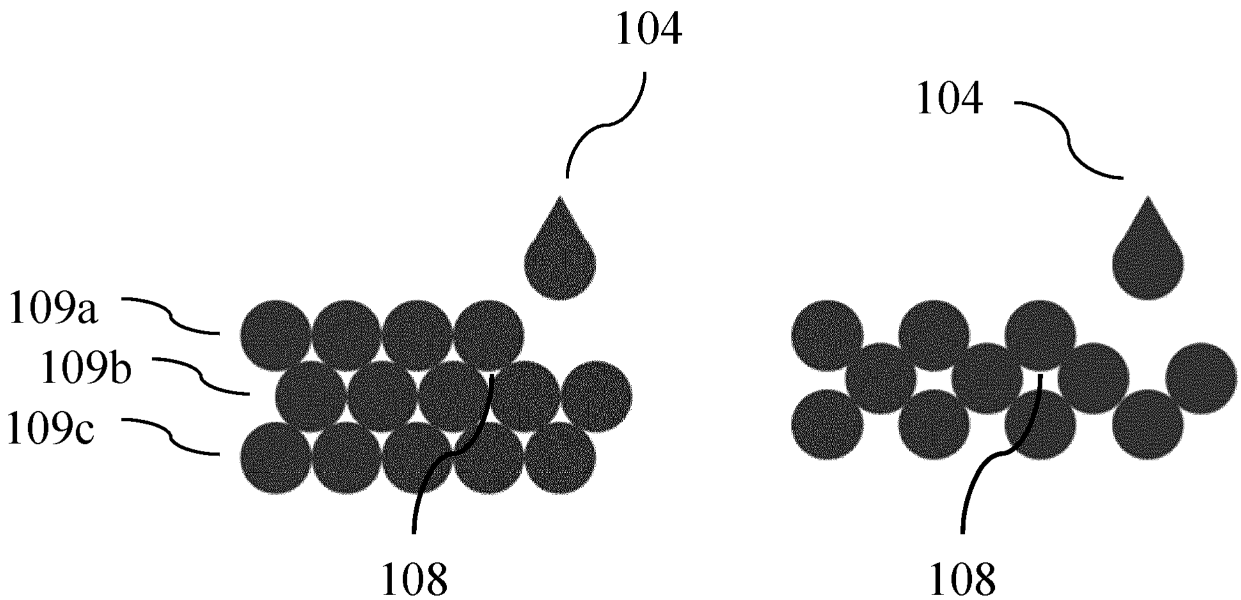

Fig. 5

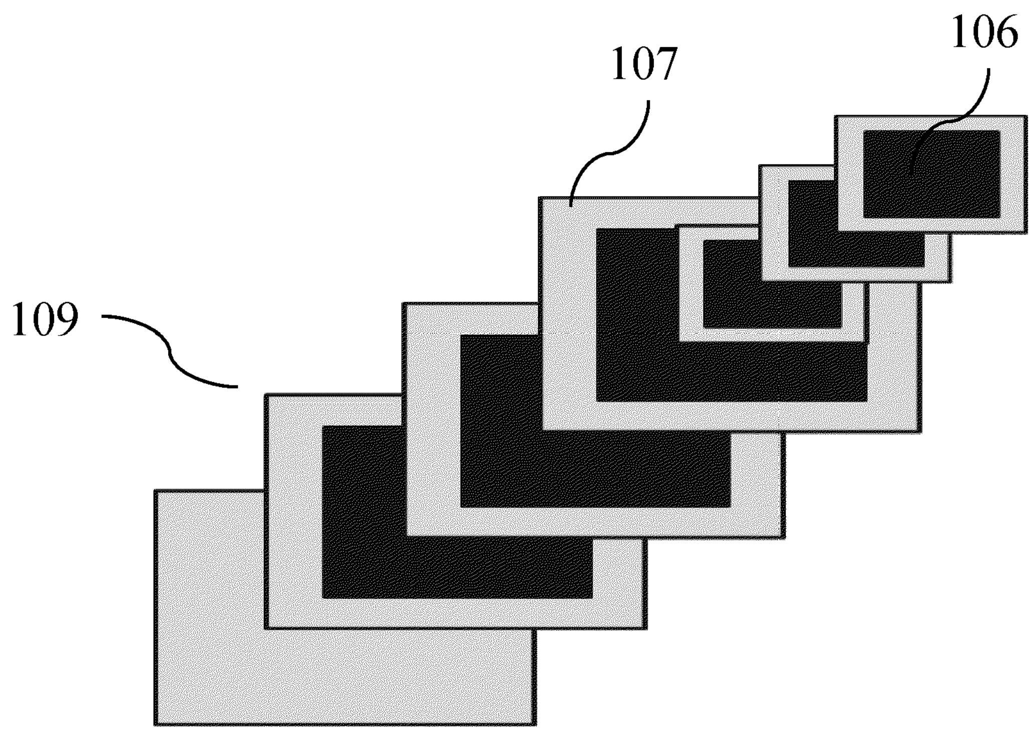

Fig. 6

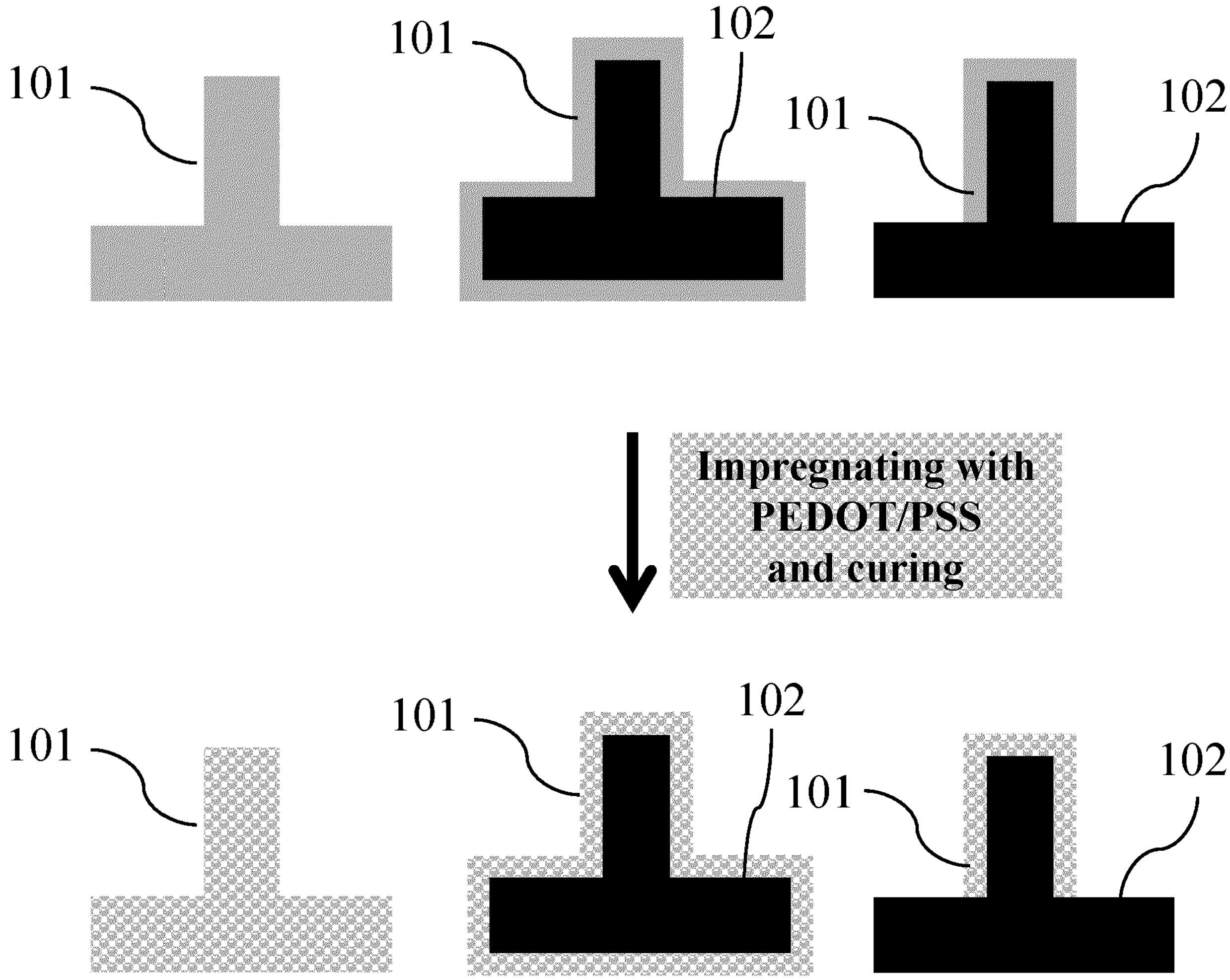
101
101
102
101
102
Impregnating with
PEDOT/PSS
and curing
101
101
102
101
102

Fig. 7

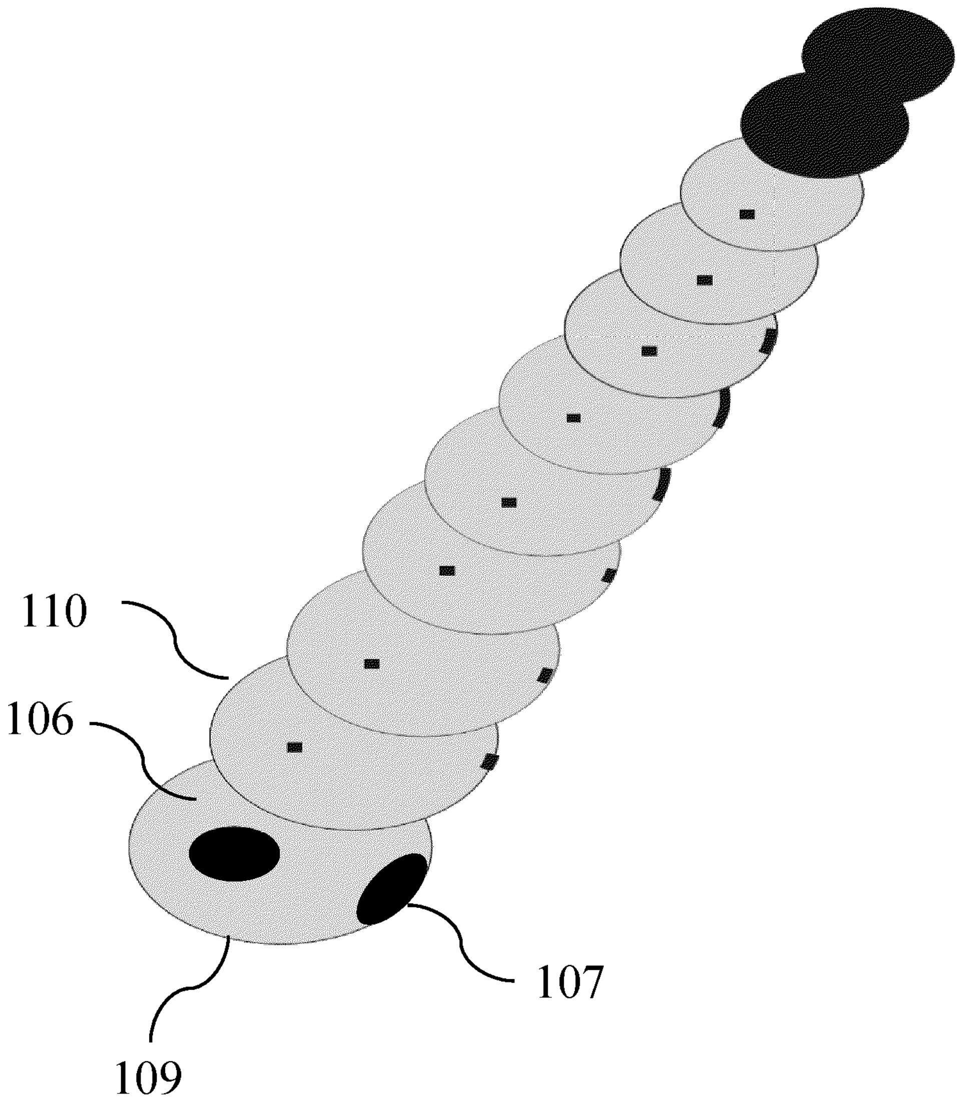

Fig. 8

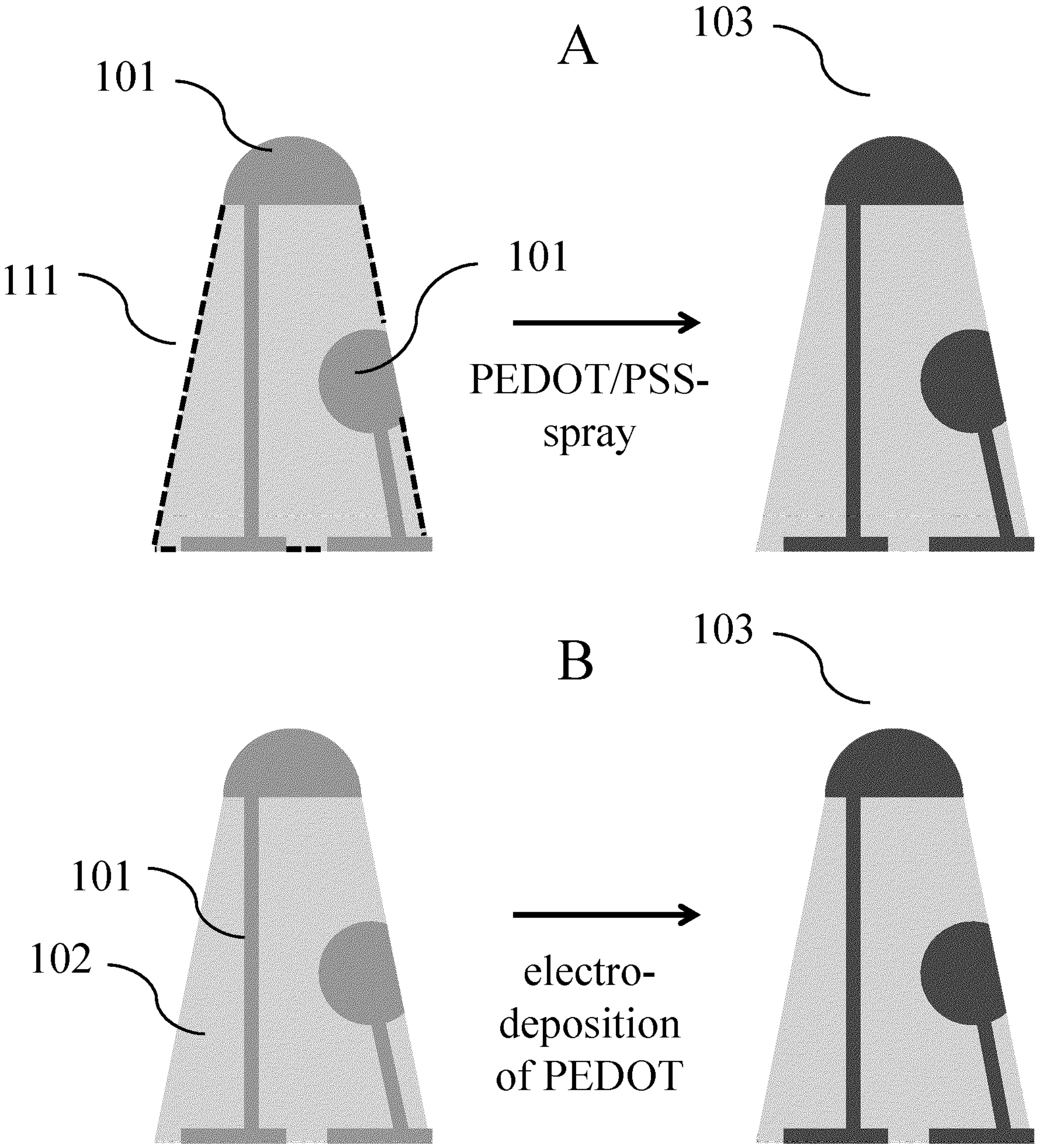
A
101
103
111
101
PEDOT/PSS-
spray
B
103
101
102
electro-
deposition
of PEDOT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9050451 B1
- US 7136691 B2
- DE 102008050932 A1
- US 9682225 B2
- WO 2019129391 A1
- EP 3400865 A1
- WO 2012041507 A1
- US 7267794 B
- US 7276361 B
- US 7208535 B
- US 2012156319 A1


### Non-patent literature cited in the description


- **LELEUX et al.** *Adv. Healthcare Mater,* 2014, vol. 3, 1377-1380